# EUROPEAN PATENT SPECIFICATION

(11) **EP 0 991 942 B1**
(45) Date of publication and mention of the grant of the patent: **13.10.2004**
(21) Application number: 98932106.2
(22) Date of filing: 02.06.1998
(51) Int. Cl.: G01N 33/50

(54) **PESTICIDE SCREENING SYSTEM**
PESTIZIDESCREENINGSVERFAHREN
SYSTEME DE SELECTION DE PESTICIDES

(30) Priority: 04.06.1997 EP 97810345; 03.02.1998 GB 9802250
(43) Date of publication of application: 12.04.2000
(73) Proprietor: Syngenta Participations AG, 4058 Basel (CH)
(72) Inventor: BOUTSALIS, Peter, CH-4422 Arisdorf (CH)
(86) International application number: PCT/EP1998/003279
(87) International publication number: WO 1998/055860

(56) References cited:
- EP-A- 0 392 225
- EP-A- 0 532 206
- WO-A-96/10083
- WO-A-97/20209
- K. GROSSMANN ET AL.: "Heterotrophic plant cell suspension cultures for monitoring biological activity in agrochemical research. Comparison with screens using algae, germinating seeds and whole plants." PESTICIDE SCIENCE, vol. 35, no. 2, 1992, pages 283-289, XP002045270 OXFORD UK
- J. GRESSEL: "Plant tissue culture systems for screening of plant growth regulators: hormones, herbicides and natural phytotoxins" ADVANCES IN CELL CULTURE, vol. 3, 1984, pages 93-181, XP002045492 NEW YORK NY USA

## Description

The present invention is in the field of plant cultivation and pesticide screening. In particular, the invention relates to a quick and easy method for testing asexually propagated plants within a pesticide screening program.

Currently, most pesticide screens are operated with whole plants propagated from plant seed or with plant tissue culture material. For example, the most common weed control technique used in agriculture today is control by herbicides. In the herbicide field, there is, therefore, a need to have a testing system available for screening weed plants for herbicide resistance to be able to quickly determine, whether the weeds are 'resistant' to the herbicide or whether some 'other' factor is involved which affects (reduces) the performance of the herbicide. Such 'other' factors may involve environmental conditions affecting the plant such that herbicide efficacy is reduced.

In order to be able to decide whether the observed reduction in herbicide performance is caused by a resistance trait or involve other factors as defined herein before, the supposedly 'resistant' weed plants are to be examined in some more detail. The current state of technology available to test weeds for their herbicide resistance status is quite poor and time consuming. The tests currently available are:
(1) Seed collection- the 'most widely' used system currently in operation. This system involves the collection of seeds from the suspected resistant weeds and then screening (herbicide spraying) plants (growing in pots containing soil) germinated from the collected seeds. Problems associated with this test include:
   (a) the suspected resistant plants are not directly tested but their progeny which may be genetically different for the herbicide resistant trait,
   (b) seed may be also collected unknowingly from plants that germinated after herbicide application which may be herbicide sensitive (susceptible) and thus confuse the test results.
   (c) seed dormancy is often a problem. If there isn't at least 70% germination, then whether the plants tested from the germinated seeds are representative of the entire seed collection for resistance is unknown,
   (d) the time required to test the weeds can be several months after seeds are collected and therefore the farmer does not have an answer on the resistance status of the weeds before planning the following seasons herbicide budget.
(2) Field test re-spraying- this system is mainly being trailed in Australia. It involves (after herbicide resistance is suspected) conducting a small herbicide field trial by re-spraying plots with herbicides in the field in the current season. Problems associated with this test include:
   (a) the trials are subject to environmental conditions which may affect the results
   (b) they are also labor and time intensive
   (c) experimental errors may occur in conducting and interpreting the trial
   (d) weed growth stage may be too late, even for higher herbicide rates
(3) Agar tests- in this system, seeds are germinated on agar containing herbicide in Petri dishes. Problems associated with this test include, as well as those associated with Test 1:
   (a) not all herbicides can be used in this system
   (b) interpretation of results is often difficult
Tests 1 and 2, although having associated disadvantages, utilize herbicide spraying similar to the field operation.
In addition, there are tests available, that use specific biochemical or molecular techniques for herbicide resistance testing of field collected weeds. These test are dependent on an operational and well equipped laboratory. A further disadvantage, which makes these tests unsuitable for large scale herbicide resistance testing, is that they test for one specific mechanism of resistance. However, due to genetic diversity, weeds (even within a single field) may possess more that one mechanism endowing herbicide resistance. Furthermore, there may be multiple mechanisms even within a single plant.
Thus currently, testing of weed populations for herbicide resistance can only be accurately conducted by spraying herbicides, just as it is done in the field. This is because only spraying accounts for all possible mechanisms of herbicide resistance, both known and possibly unknown.

Similar problems are also prevalent in the fungicide and insecticide field. Here it is often difficult to decide whether a resistance phenotype exhibited by a crop or non-crop plant is due to a resistance trait or caused by some 'other' factors. This especially applies to transgenic plants having been transformed with a certain resistance trait, where a quick and reliable decision as to whether the observed resistance phenotype is likely to represent a true transformant is often desirable.

Furthermore, treatment of a crop plant with a pesticide may result in undesirable plant damage. In such a situation it would be highly desirable to know whether the observed damage is caused by the pesticide action or whether some 'other factors' are involved that affect the performance of the crop plant such as, for example, contamination of the pesticide preparation or the soil with plant toxic substances, etc.

In consideration of the above outlined problems it is one of the main objectives of the present invention to provide a quick and easy system for testing plants that leads to reliable results, but does not encounter the many disadvantages of the systems currently available in the art and can thus be suitably used within a pesticide screening program. This objective could be surprisingly met by applying techniques well known in the art.

It is thus main object of the invention to provide a method for testing progeny plants, comprising
(a) asexually propagating progeny plant(s) from a mother plant, preferably a transgenic plant, but especially a weed or crop plant by cutting a segment from a mother plant, said segment comprising a meristematic region which is sufficiently intact to have retained its capability of regenerating a complete and morphologically normal plant, and directly regenerating said segment;
(b) incorporating the so obtained progeny plant into a plant screening program; and
(c) monitoring the growth of the progeny plant.

In a preferred embodiment of the invention, the propagation step is accomplished by
(a) cutting a short segment from a mother plant preferably a transgenic plant, but especially a weed or crop plant such that said segment is capable of directly regenerating into a whole and morphologically normal plant, preferably a region that contains a high amount of actively dividing cells, most preferably a region that comprises meristematic cells;
(b) transferring said excised segment to a suitable anchorage material; and
(c) directly regenerating said transferred segment into a whole and morphologically normal plant.

In a further preferred embodiment of the invention, the excised plant segment comprises a short root and shoot fragment.

The method according to the invention can preferably be used within a high through-put format.

The invention further encompasses a method comprising
(a) cutting a short segment from a mother plant, preferably a transgenic plant, but especially a weed or crop plant such that said segment comprises a meristematic region which is sufficiently intact to be capable of directly regenerating into a whole and morphologically normal plant,
(b) dipping said segment into a known concentration(s) of a pesticide-containing solution;
(c) transferring the so treated plant explants to a suitable anchorage material;
(d) directly regenerating said explant into a whole and morphologically normal plant; and
(e) monitoring the growth of the progeny plant.

It is a further objective of the invention to provide a method of rescuing plants showing an interesting trait or property after treatment with a pesticide for further investigation comprising
(a) asexually propagating progeny plant(s) from a treated mother plant, preferably a transgenic plant, but especially a weed or crop plant by cutting a segment from said mother plant, said segment comprising a meristematic region which is sufficiently intact to have retained its capability of regenerating a complete and morphologically normal plant, and directly regenerating said segment;
(b) incorporating the so obtained progeny plant into a plant screening program; and
(c) monitoring the growth of the progeny plant.

In a preferred embodiment of the invention the propagation step is accomplished by
(a) cutting a short segment from a treated mother plant such that said segment comprises a meristematic region which is sufficiently intact to be capable of directly regenerating into a whole and morphologically normal plant,
(b) transferring said excised segment to a suitable anchorage material; and
(c) directly regenerating said transferred segment into a whole and morphologically normal plant.

The invention further encompasses a method comprising
(a) cutting a short segment from a treated mother plant, preferably a transgenic plant, but especially a weed or crop plant such that said segment comprises a meristematic region which is sufficiently intact to be capable of directly regenerating into a whole and morphologically normal plant,
(b) dipping said segment into a known concentration(s) of a pesticide-containing solution, preferably a solution containing a pesticide selected from the group consisting of a herbicide, an insecticide and a fungicide;
(c) transferring the so treated plant explants to a suitable anchorage material;
(d) directly regenerating said explant into a whole and morphologically normal plant; and
(e) monitoring the growth of the progeny plant.

Also comprised by the invention is a method for determining whether a resistance phenotype observed in a plant is due to a resistance trait or caused by other factors, comprising
(a) collecting the phenotypically resistant plant, preferably a transgenic plant, but especially a weed or crop plant,
(b) asexually propagating progeny plant(s) from said resistant plant by cutting a segment from said resistant plant, said segment comprising a meristematic region which is sufficiently intact to have retained its capability of regenerating a complete and morphologically normal plant, and directly regenerating said segment;
(c) incorporating the so obtained progeny plant into a plant screening program; and
(d) monitoring the growth of the progeny plant.
or, alternatively
(a) collecting the phenotypically resistant plant, preferably a transgenic plant, but especially a weed or crop plant
(b) cutting a short segment from said plant such that said segment comprises a meristematic region which is sufficiently intact to be capable of directly regenerating into a whole and morphologically normal plant,
(c) dipping said segment into a known concentration(s) of a pesticide- containing solution, preferably a solution containing a pesticide selected from the group consisting of a herbicide, an insecticide and a fungicide;
(d) transferring the so treated plant explants to a suitable anchorage material;
(e) directly regenerating said explant into a whole and morphologically normal plant; and
(f) monitoring the growth of the progeny plant.

In particular, the present invention provides a quick and easy method for testing progeny plants that have been propagated asexually from a mother plant. The progeny plants to be tested are propagated asexually from a mother plant; preferably by cutting a short segment from the mother plant which segment is capable of directly regenerating into a whole and morphologically nonnal plant. Preferred within the scope of the inventions are plant segments which comprise regions that contain a high amount of actively dividing cells. Especially preferred are plant segments comprising one or more regions containing a high amount of meristematic cells such as, for example, a basal segment of a monocotyledonous plant including a short root and stem, or a segment of a broad-leaved (dicotyledon) plant comprising a short above-ground nodal section. In rhizomeaceous plants those segments are preferred that comprise a short rhizome segment containing roots.

The minimal size of the segment used for regenerating a whole plant should be such that the meristematic region contained in said segment is sufficiently intact to have retained its capability of regenerating a complete and morphologically normal plant. In order to speed up plant regeneration and development it may be advantageous if the plant segment excised from the mother plant still contains a short root or shoot fragment.

No special equipment is needed for cutting the segments from the plant; in principle, any household cutting device can be used for.that purpose. The device should be constituted such that serious damage to plant tissue, which increases the likelihood of contamination, can be avoided. Preferred are, therefore, clean sharp scissors or a sharp knife or scalpel. The cutting devices to be used in the present invention are preferably cleaned prior to being used, for example by rinsing in clean tap or distilled water to minimize disease transfer between plants.

The so obtained plant segments are subsequently transferred to a suitable plant anchorage material such as, for example, a culture medium commonly employed in plant cultivation or any other suitable plant anchorage material that supports the development and growth of the plant explant. If a culture medium is used, precautions are to be taken to avoid contamination of the medium by outdoor contaminants. For example, the plant material may be surface sterilized prior to its use by incubating it with a 5% to 10% Clorox solution for about 5-10 minutes, preferably followed by several washes with sterile distilled water. If an inert plant anchorage material is used such as vermiculite, perlite or plastic beads, plant development and growth supporting and promoting agents are to be provided to the plant in a manner that it can be readily accessed by the plant explant in order to support a normal plant growth and development. Ready-to-use nutrient solutions that can be used within the scope of the invention are offered commercially and are well known to those skilled in the art of plant cultivation. For example, nutrition in the form of a complete commercial fertilizer is adequate for the purpose of the present invention. Any complete soluble fertilizer or even N:P:K soluble fertilizer will suffice since the duration of the experiment is rarely more that one month. Plant promoting agents which stimulate root and shoot formation of the plant explant and can be suitably used for the purpose of the invention are also well known to those skilled in the art and comprise, for example, GA3, NAA and 6BAP are hormones widely used in tissue culture for root and shoot stimulation.

In the easiest and most convenient embodiment the plant segment is transferred into any general potting soil, which optionally may contain added fertilizer, or even into field soil. Experiments have shown that *Alopecurus myosuroides* explants grew equally well in vermiculite, sand and in loam. In field soil, all that is necessary is for the plant segments to be watered as required, with no extra fertilizer necessary. It may be advantageous to sterilize the soil prior to its use in order to avoid contamination by outdoor contaminants.

This can be done by applying conventional means such as, for example, heat, preferably in combination with high pressure.

The plant explants are then cultivated under conditions that allow the regeneration of whole and fully developed plants. Optimal results are to be obtained by cultivating said explants under standardized conditions within a temperature-controlled green-house or a growth chamber. Standardized culturing conditions are preferable in order to speed up plant development and growth, but are not a prerequisite for operating the method according to the invention. Plant cultivation can be done in a temperature range of between 12°C and 28°C depending on the plant species used as a source for the explant to be regenerated. Preferred is a growing temperature in the range of between 15°C and 25°C. However, if speed of getting a result is not critical, then the explants can also be grown outside. Lower temperatures mean that pesticide effects will be slower, and if high temperatures are prevalent, then correct moisture levels must be maintained.

A person experienced in the field of plant cultivation and regeneration would certainly appreciate that dependent on the plant species used certain adaptations of the procedure outlined above in general terms might be necessary to meet the specific requirements of the respective plant species. The specific measures to be taken in order to guarantee an orderly growth and development of the plant are well known to those skilled in the pertinent art.

The regenerated plantlets having been developed from the previously excised plant explants can then be incorporated into any of the commonly applied pesticide screening programs. Owing to the simplicity and easiness of the system according to the invention, the time range between the cutting of the plant segments and the principle availability of the plants regenerated from said segments to become incorporated into a screening program is extremely short. Depending on the plant species involved it may be between about 1 day to about 12 weeks, preferably 5 days to about 10 weeks, more preferably between about 7 days to about 5 weeks and most preferable between about 9 days to about 14 days.

The screening assay to be applied to the regenerated plantlets may vary depending on the underlying question to be answered. If the question is, for example, concerned with a herbicide resistance issue, the plantlets are subjected to spray program. The pots containing the plantlets in one of the previously described anchorage materials can be sprayed in a spray chamber with the herbicide to be tested without the necessity of taking any preliminary measures.

Herbicides that can be tested within the method for testing progeny plants according to the invention comprise, but are not limited to, 2 4-D, 2 4-DB, acetochlor, acifluorfen, alachlor, ametryne, amidosulfuron, amitrol, atrazine, aziprotryn, BAY FOE 5043, bensutfuron, bentazone, bifenox, bromofenoxim, bromoxynil, butylate, chlorbromuron, chloridazon, chloridazone, chlorimuron-ethyl, chlorsulfuron, chlortoluron, cinosulfuron, clethodim, clodinafop optionally in combination with cloquintocet, clomazone, clopyralid, cloransulam, cyanazine, cycloate, cycloxydim, desmedipham, desmetryn, dicamba, diclofop, difenzoquat , diflufenican , diflufenzopyr, dimethachlor, dimethametryn, dimethenamide and its S-enantiomer, dipropertryn, diquat, diuron, DTPA NaFE, EDDHA NaFe, endothal, EPTC, ethaffluralin, ethametsulfuron, ethofumesate, fenclorim, fenoxaprop, flamprop, fluazifop, flumetsulam, fluometuron, fluorchloridone, fluoxypyr, fluthiacet-methyl, fomesafen, glufosinate, glyphosate, halosulfuron, haloxyfop, imazamethabenz, imazapyr, imazaquin, imazethapyr, ioxynil, isoproturon, karbutilate, lactofen, lenacil, linuron, MCPA, metamitron, methabenzthiazuron, metobromuron, metolachlor and its S-enantiomer optionally in combination with benoxacor or oxim safener, metosulam, metoxuron, metribuzin, metribuzin, metsulfuron-methyl, molinate, nicosulfuron, norflurazon, oxabetrinil, oxasulfuron, paraquat, pebulate, pendimethalin, phenmedipham, picloram, piperophos, pretilachlor, primisulfuron, prodiamine, prometon, prometryne, propachlor, propanil, propaquizafop, propazin, prosulfuron, pyridate, pyrithiobac, quinmerac, quizalofop, rimsulfuron, sethoxydim, simazine, simetryn, sulcotrione, sulfometuron-methyl, sulfosate, tebutam, terbacil, terbumeton, terbumeton, terbuthylazine, terbutryn, thiazafluron, thiazafluron, thifensulfuron, tralkoxydim, triallate, triasulfuron, tribenuron-methyl, trifluralin and trinexapac-ethyl. It is understood that also known mixtures of two and more of the above-mentioned herbicides as well as known mixtures thereof with safeners can be used in the inventive method. A number of such mixtures are known in particular from the patent literature.

It is to be understood that the method for testing progeny plants according to the invention is in no way confined to the herbicide area, but can be used wherever there is a need for a quick and reliable system for testing asexually propagated plants for resistance including insecticide and fungicide resistance, pathogen resistance, and the like.

Depending on the area where the method according to the invention is to be used, the target plants to which said system can be potentially applied, may vary. In the herbicide area, the main focus is likely to be on testing weed plants for herbicide resistance symptoms, as discussed herein previously.

Weed plants that can be suitably screened for herbicide resistance within the scope of the method according to the invention comprises, without being limited to *Echinochloa crus-galli* (Barnyardgrass), *Setaria vericillata* (Bristly Foxtail), *Opuntia spp.* (Cactus), *Bromus fectorum* (Downy Brome), *Festuca arundinacea* (Fescue), *Digitalis purpurea* (Foxglove), *Hordeum jubatum* (Foxtail Barley), *Setaria faberi* (Giant Foxtail), *Setaria viridis* (Giant Green Foxtail), *Digitaria sanguinalis* (Hairy Crabgrass), *Sorghastrum nutans* (Indiangrass), *Sorghum halepense* (Johnsongrass), *Polygonum aviculare* (Knotweed), *Polygdhum persicaria* (Ladysthumb Smartweed ), *Alopecurus pratensis* (Meadow Foxtail), *Dactylis glomerata* (Orchardgrass), *Polygonum pensylvanicum* (Pennsylvania Smartweed), *Thalspi arvense* (Pennycress), *Amaranthus spp.* (Pigweed), *Amaranthus spinosus* (Prickly Pigweed), *Amaranthus blitoides* (Prostrate Pigweed), *Euphorbia spp.* (Prostrate Spurge), *Agropyron repens* (Quackgrass), *Amaranthus palmeri* (Redroot Pigweed), *Agrostis alba* (Redtop), *Phalaris arundinacea* (Reed Canarygrass), *Juncus spp.* (Rush), *Sorghum bicolor* (Shattercane/Wild Cane), *Bromus inermis* (Smooth Bromegrass), *Digitaria ischaemum* (Smooth Crabgrass), *Amaranthus hybridus* (Smooth Pigweed), *Amaranthus hybridus*/*retroflexus* (Smooth/Redroot Pigweed), *Polygonum coccineum* (Swamp Smartweed), *Panicum virgatum* (Switchgrass), *Amaranthus tuberculatus* (Tall Waterhemp), *Campsis radicans* (Trumpet Creeper), *Amaranthus albus* (Tumble Pigweed), *Hibiscus trionum* (Venice Mallow), *Parthenocissus quinquefolia* (Virginia Creeper), *Lepidium virginicum* (Virginia Pepperweed), *Calla palustris* (Water-lilly), *Ellisia nyctelea* (Waterpod), *Trifolium repense* (White Clover), *Polygonum convolvulus* (Wild Buckwheat), *Vitis spp.* (Wild Grape), *Panicum miliaceum* (Wild Proso Millet), *Panicum capillare* (Witchgrass), *Setaria lutescens* (Yellow Foxtail), *Cyperus esculentus* (Yellow Nutsedge), *Trifolium agrarium* (Yellow Clover), *Yucca glauca* (Yucca), *Aegilops spp., Agropyron spp., Allium spp., Alopecurus spp., Avena spp*., *Briza spp*., *Brachiaria spp*., *Bromus spp*., *Cynodon spp., Cyperus spp., Dactylis spp*., *Dactylutenium spp*., *Danthonia spp., Datura spp., Digitaria spp., Echinochloa spp., Eleusine spp*., *Ergrostis spp., Eriochloa spp., Festuca spp*., *Holcus spp*., *Hordeum spp*., *Juncus spp*., *Lolium spp., Panicum spp., Paspalum spp*., *Pennisetum spp*., *Phalaris spp., Phragmites spp., Poa spp., Polypogon spp*., *Puccinellia spp., Setaria spp*., *Sorghum spp., Vulpia spp.*

Preferred are monocotyledonous plants such as *Allium* spp., *Cyperus* spp., and *Juncus* spp., more preferably graminaceous monocots, and most preferably *Agropyron* spp., *Alopecurus* spp., *Avena* spp., *Bromus* spp., *Dactylis* spp., *Digitaria* spp., *Echinochloa* spp., *Festuca* spp., *Hordeum* spp., *Lolium* spp., *Panicum* spp., *Phalaris* spp., Poa spp., *Setaria* spp., *Sorghum* spp., *Vulpia* spp. Also embodied by the present invention is the use of dicotyledonous plants, preferably broad-leaved (dicotyledonous) plants such as *Amaranthus* spp. *Chenopodium* spp and *Atriplex* spp. A third group of plants that can be suitably used within the method according to the invention are rhizomeaceous plants such as some *Polygonum* spp.

It is to be emphasized that the present invention is in no way confined to the screening of weed plants for herbicide resistance. There might very well be situations, where it is desirable to have a quick and reliable method available for screening crop or non-crop plants in order to determine whether resistance symptoms observed in the field or the green house can be confirmed in genetically identical or at least almost identical progeny plants. Even in the opposite case, where a crop plant known to be resistant to a specific disease shows unexpected disease symptoms, it might be desirable to have a quick and reliable method available in order to determine whether the observed symptoms are caused by a loss of the resistance trait or whether some 'other' factor is involved which affects (reduces) the performance of the plant.

In using the process according to the present invention, plants showing an interesting trait or property after treatment with a pesticide can be rescued for further investigation. In case of plants forming tillers, plants rescued from said tillers may be used for genetic studies because, tillers are supposed to be genetically identical with respect to a particular trait.

For example, plants, which may be either crop or weed plants, that show unexpected resistance properties in the field or green-house, but especially resistance towards pathogenic fungi or insects, may be collected. Plants are rescued from the collected material by asexually propagating progeny plants, preferably by cutting a short segment from the previously collected plant material such that it is capable of directly regenerating into a whole plant, as described herein before. The so obtained plant segment is then transferred to a suitable anchorage material but preferably to soil and regenerated into a whole and morphologically normal plant. This plant can then be introduced into one of the screening programs that are commonly used in the respective research area and the performance of said plant be monitored.

In case of an observed insect resistance, said screening program may involve an insect feeding assay, whereas in case of an observed fungal resistance, the so produced plants may be incorporated into a spray program where the plant is subjected to one or more spore-containing solutions representing different plant fungal pathogens.

Depending on the performance of the plant in the respective assay it can immediately be determined whether the previously observed resistance is likely to be due to a resistance trait or caused by some 'other' factor as discussed herein before.

The method according to the invention can also be suitably used for answering the question whether plant damage observed after treating the plant, preferably a crop plant, with a pesticide is likely to be caused by the action of a pesticide having been applied to the plant or by some 'other' factors such as, for example, contamination of the pesticidal preparation with undesired herbicides, adverse environmental conditions affecting the plant such that it becomes more susceptible to the applied compound, residual effects caused by contaminated soil, etc.

In that case plants are rescued from the damaged plants by asexually propagating progeny plants, preferably by cutting a short segment from the previously collected plant material such that it is capable of directly regenerating into a whole plant, as described herein before. The so obtained plant segment is then transferred to a suitable anchorage material but preferably to soil and regenerated into a whole and morphologically normal plant. This plant can then be introduced into a spray program. The trays containing the plantlets in one of the previously described anchorage materials can be sprayed in a spray chamber with the respective pesticide to be tested without the necessity of taking any preliminary measures. The performance of the treated plants is monitored for a given period of time and, depending of the results obtained, a decision can be made as to whether the observed damaging effects are likely to be caused by the action of the pesticide or by some 'other' factors.

Further, it is now possible to quickly determine the lifetime of a pesticidal compound within a plant after treatment of the plant with a solution containing said pesticidal compound. Plants are rescued from the treated material by asexually propagating progeny plants, preferably by cutting a short segment from the previously treated plant material which is capable of directly regenerating into a whole plant, as described herein before. The so obtained plant segment is then transferred to a suitable anchorage material but preferably to soil and regenerated into a whole and morphologically normal plant. This plant can then be introduced into one of the testing systems that are commonly used in the respective research area.

In case of plants having been treated with an insecticidal compound, this may be an insect feeding assay, whereas in case of a plant having been treated with a fungicide compound, the so produced progeny plants may be incorporated into a spray program where the plant is subjected to one or more spore-containing solutions representing different plant fungal pathogens. Dependent on the results obtained it can then be determined, whether the pesticidal compound stayed with the progeny plant or was already converted into an inactive form.

Insecticides that can be used within the method for testing progeny plants according to the invention comprise, but are not limited to: Aldicarb; Azinphos-methyl; Benfuracarb; Bifenthrin; Buprofezin; Carbofuran; Dibutylaminothio; Cartap; Chlorfluazuron; Chlorpyrifos; Cyfluthrin;Lambda-Cyhalothrin; Alpha-cypermethrin; zeta-Cypermethrin; Deltamethrin; Diflubenzuron; Endosulfan; Ethiofencarb; Fenitrothion; Fenobucarb; Fenvalerate; Formothion; Methiocarb; Heptenophos; Imidacloprid; Isoprocarb; Methamidophos; Methomyl; Mevinphos; Parathion; Parathion-methyl; Phosatone; Pirimicarb; Propoxur; Teflubenzuron; Terbufos; Triazamate; Abamectin; Fenobucarb; Tebufenozide; Fipronil; beta-Cyfluthrin; Silafluofen; Fenpyroximate; Pyridaben; Fenazaquin; Pyriproxyfen; Pyrimidifen; Nitenpyram; NI-25, Acetamiprid; Avermectin B₁ (Abamectin); AC 303 630; Acephat; Acrinathrin; Alanycarb; Alphamethrin; Amitraz; AZ 60541; Azinphos A; Azinphos M; Azocyclotin; Bendiocarb; Bensultap; Betacyfluthrin; BPMC; Brofenprox; Bromophos A; Bufencarb; Butocarboxin; Butylpyridaben; Cadusafos; Carbaryl; Carbopheno-thion; Chloethocarb; Chlorethoxyfos; Chlormephos; Cis-Res-methrin; Clocythrin; Clofentezin; Cyanophos; Cycloprothrin; Cyhexatin; Demeton M; Demeton S; Demeton-S-methyl; Dichlofenthion; Didiphos; Diethion; Dimethoat; Dimethylvinphos; Dioxathion; Edifenphos; Emamectin; Esfenvalerat; Ethion; Ethofenprox; Ethoprophos; Etrimphos; Fenamiphos; Fenbutatinoxid; Fenothiocarb; Fenpropathrin; Fenpyrad; Fenthion; Fluazinam; Flucyctoxuron; Flucythrinat; Flufenoxuron; Flufenprox; Fonophos; Fosthiazat; Fubfenprox; HCH; Hexaflumuron; Hexythiazox; Iprobenfos; Isofenphos; Isoxathion; Ivermectin; Lambda-cyhalothrin; Malathion; Mecarbam; Mesulfenphos; Metaldehyd; Metolcarb; Milbemectin; Moxidectin; Naled;NC 184; Omethoat; Oxamyl; Oxydeme- thon M; Oxydeprofos; Permethrin; Phenthoat; Phorat; Phosmet; Phoxim; Pirimiphos M; Pirimiphos A; Promecarb; Propaphos; Prothiofos; Prothoat; Pyrachlophos; Pyrada-phenthion; Pyresmethrin: Pyrethrum; RH 5992; Salithion; Sebufos; Sulfotep; Sulprofos; Tebufenpyrad; Tebupirimphos; Tefluthrin; Temephos; Terbam; Tetrachlor-vinphos; Thiafenox; Thiodicarb; Thiofanox; Thionazin; Thuringiensin; Tralomethrin; Triarthen; Triazophos; Triazuron; Trichtorfon; Triflumuron; Trimethacarb; Vamidothion; Xylylcarb; YI 5301/5302; Zetamethrin; DPX-MP062; RH-2485; D 2341; oderXMC (3,5,-Xy-lyl Methylcarbamat), Azamethiphos; Chlorfenvinphos; Cypermethrin, Cypermethrin high-cis; Cyromazin; Diafenthiuron; Diazinon; Dichlorvos; Dicrotophos, Dicyclanil; Fenoxycarb; Fluazuron; Furathiocarb; Isazofos; Jodfenphos; Kinoprene; Lufenuron; Methacriphos; Methidathion; Monocrotophos; Phosphamidon; Profenofos; Diofenolan; eine Substanz erhältlich aus dem Bacillus thuringiensis Stamm GC91 oder aus NCTC1 1821; Pymetrozine; Bromopropylate; Methoprene; Disulfuron; Quinatphos; Tau-Fluvalinat; Thiocyclam; oder Thiometon.

Fungicides that can be used within the method for testing progeny plants according to the invention comprise, but are not limited to: furalaxyl, metalaxyl, R-metataxyl, benzoylprop ethyl, benalaxyl, oxadixyl, flamprop methyl, ofurace, difenoconazole, etaconazole, propiconazole, penconazole, triadimefon, triadimenol, epoxiconazole, tebuconazole, bromuconazole, fenbuconazole, cyproconazole, tetraoonazol, metconazol, flusilazol, diniconazol, triticonazole, uniconazole, midobutanil, fluquinconazole, kresoxim-methyl, azoxystrobin, methoxyimino-{2-[1-(3-trifluoromethyl-phenyl)- ethylideneaminooxymethyl]-phenyl}-acetic acid methyl ester, aldimorph, tridemorph, dimethomorph, fenpropimorph, fenpropidin, bromoxynil, carboxin, prachloraz, propargite, dicamba, fenpiclonil, fludioxonil, pymetrozine, pyrifenox, pyriproxyfen, fluazinam, chlorothalonil, pyroquilon, bacillus thuringiensis, captan, captafol, folpet, mancozeb, maneb, zineb, Cu-compounds, ethyrimol, fenarimol, nuarimol, cymoxanil, imazalil, pyrazophos, thiabendazol, triforine, tricyclazole, iprodione, carboxine, thiram, acibenzolar-s-methyl, famoxadone, quinoxyfen, sptroxamin, fenhexamid.

In an analogous manner plants treated with other pesticidal compounds can be tested within the method for testing progeny plants according to the invention such as, for example, molluscicides, nematicides, miticides, etc.

In a further embodiment of the invention, plants, which have been genetically engineered to contain a pesticide resistance trait can be suitably used within the process according to the invention. For example, plants transformed with a resistance gene showing a resistance phenotype can be rescued and further investigated thereby confirming that the individual plant or plants are truly resistant.

Structural genes which are preferably used to confer a particular resistance trait to a plant are those which code for proteins which are able to protect plants against pathogens (for example phytopathogenic fungi, bacteria, viruses, etc.), herbicides (for example triazines, sulfonylureas, imidazolinones, triazolepyrimidines, bialaphos, glyophosates, etc.), fungicides, insecticides or disadvantageous environmental influences (for example heat, cold, wind, unfavorable soil conditions, moisture, dryness, etc.).

Within the scope of this invention, the use of transformed plants comprising structural genes associated with the control of plant pathogens and parasites are particularly preferred.

For example, resistance towards insects can be transferred by a gene which codes for a polypeptide which is toxic for insects and/or their larvae, for example the crystalline protein of *Bacillus thuringiensis*. Such genes are known and described, for example, in US-P 4,865,981, US-P 4,996,155, WO 89/07605, EP 213;318 and EP 186,379. A further class of vegetative insecticidal protein encoding DNA sequences is described in WO 94/21795 and WO 96/10083.

The protease inhibitors are a second class of proteins which mediate insect resistance. Protease inhibitors form a normal constituent of plant storage structures and for this reason are usually located in vacuoles or "protein bodies". Thus, it was possible to demonstrate that the Bowman-Birk protease inhibitor, which was isolated and purified from soya beans, inhibits the intestinal protease of Tenebrio larvae (Birk Y *et al* (1963) Biochim. Biophys. Acta, 67: 326-328). The gene, which codes for a trypsin inhibitor from the common cowpea, is described in Hilder *et al* (1987) Nature, 330: 160-163.

Within the scope of the present invention, plants comprising any *Bacillus thuringiensis* crystal protein, vegetative protein, protease inhibitor or any other insecticidal protein-encoding DNA sequences can be used within the method according to the invention, irrespective of their provenance (e.g. insecticidal proteins from non-plant sources or from purely synthetic sources).

In this connection, mention must also be made of hydrolytic enzymes which are either able on their own to bring about degradation of the cell walls of plant pathogens or else are at least able to support this process, in a synergistic sense, in combination with other substances.

For example, the majority of insects possess a cuticular skeleton in which chitin micelles, layered in a lamellar fashion, are embedded in a ground substance. Numerous plant-pathogenic fungi, for example the basidiomycetes (smut and rust fungi), ascomycetes and *Fungi imperfecti* (including *Alternaria* and *Bipolaris*, *Exerophilum turcicum, Colletotricum, Gleocercospora* and *Cercospora*), also contain chitin as an integral constituent of their hyphal and spore structures. Chitinase is able to inhibit the mycelial growth of certain pathogens in vitro. A plant organ or tissue which is able to express chitinase constitutively or else as a response to penetration by a pathogen can thus protect itself against attack by a large number of different fungi.

Another enzyme which plays an important part in the mechanisms by which plants defend themselves against pathogens is β-1,3-glucanase. Plants transformed with a.gene which encodes β-1,3-glucanase (GLB), preferably in combination with a gene encoding one of the previously mentioned chitin degrading enzymes are therefore preferably employed in the method according to the invention.

Plants that may be used within the scope of this invention also encompass those comprising a gene coding for the so-called lytic peptides. Lytic peptides in the broadest sense of the term are also to be understood as being compounds whose ability to penetrate, lyse or damage cell membranes is based on enzymatic activity, for example tysozymes and phospholipases. The amino acid sequences of various lytic peptides are shown in the following publications: WO89/11291; WO86/04356; WO88/05826; US4,810,777; WO89/04371.

Further plants that may be advantageously used within the method according to the invention are those comprising a class of genes coding for phospholipid transfer proteins disclosed, for example, in WO 92/20801.

Plant exhibiting an antifungal phenotype can be prepared by transforming plants with a class of genes that encode pathogenesis-related proteins [PRPs] such as PR-1 A, PR-1 B, PR-1C, PR-R major, PR-R minor, PR-P, PR-Q, PR-2, PR-2', PR-2", PR-N, PR-O, PR-O', PR-4, SAR8.2a-e, cucumber chitinase/lysozyme, cucumber basic peroxidase, tobacco basic glucanase and tobacco basic chitinase/lysozyme, tobacco acidic chitinase/lysozyme.

Examples of the above genes and proteins including chimeric genetic constructs comprising the said genes are provided in EP-A 392,225.

Of the plants being transformed with genes which can mediate resistance to herbicides, those containing genes for glyphosate tolerant 5-enolpyruvylshikimate-3-phosphate synthase (EPSP synthase) [EP-A 115 673; EP-A 409 815], acetolactate synthase (ALS) and glutamine synthase (GS), and the bar or PAT gene from streptomyces, which confer resistance to the herbicide phosphinotricin [White *et al* (1990) Nucl Acids Res 18: 1062; Spencer *et al* (1990) Theor Appl Genet 79: 625-631], are preferred. Further preferred are plants transformed with a gene encoding for an enzyme with a protoporphyrinogen oxidase(protox) activity, an adenylosuccinate synthetase activity and an adenylosuccinate lyase activity.

The method according to the invention can also be used for verifying the resistance status of plants having been transformed with a selection marker gene and showing a resistance phenotype with respect to said marker. Plants that are still capable of growing in the presence of the respective selection agent can be rescued and further investigated thereby confirming that the individual plant or plants are truly resistant.

Plants that can potentially be used within the scope of the present invention are those having been transformed with one of the selection markers used routinely in plant transformation including, but not limited to, the nptII gene, which confers resistance to kanamycin and related antibiotics [Messing & Vierra (1982) Gene 19, 259-268; Beven *et al* (1983) Nature 304, 184-187], the hph gene, which confers resistance to the antibiotic hygromycin [Blochinger & Diggelmann (1984) Mol Cell Biol 4, 2929-2931] and the dhfr gene, which confers resistance to methotrexate [Bourouis *et al* (1983) EMBO J 2(7), 1099-1104].

The method according to the invention can principally be applied to all crop plants. Preferred are monocotyledonous plants, but especially plants of the *Graminaceae* family involving , but without being limited to: *Lolium, Zea, Triticum, Triticale, Sorghum, Saccharum, Bromus, Orvzae, Avena, Hordeum, Secale* and *Setaria*.

Especially preferred are maize, wheat, barley, sorghum, rye, oats, turf grasses; corn, sweetcorn, and rice.

The method according to the invention can also be applied to dicotyledonous crop plants. Such plants include, but without being limited to, field crops, vegetables and fruits including: cotton, tobacco, sugar beet, oilseed rape, tomato, pepper, melon, lettuce, cauliflower, broccoli, cabbage, brussels sprout, sugar beet, onion, carrot, leek, cucumber, tobacco, alfalfa, aubergine, beet, broad bean, celery, chicory, cow pea, endive, gourd, groundnut, papaya, pea, peanut, pineapple, potato, safflower, snap bean, spinach, soybean, squashes, sunflower, water-melon, and the like; and ornamental crops including Impatiens, *Begonia, Petunia, Pelargonium, Viola, Cyclamen, Venbena, Vinca, Tagetes, Primula, Saint Paulia, Ageratum, Amaranthus, Anthirrhinum, Aquilegia, Chrysanthemum, Cineraria, Clover, Cosmo, Cowpea, Dahlia, Datura, Delphinium, Gerbera, Gladiolus, Gloxinia, Hippeastrum, Mesembryanthemum, Salpiglossis, Zinnia,* and the like.

In a specific embodiment of the invention stem explants are cut from a mother plant, as described herein before and, within a short while, that is preferably within less than about 1 hour after the excision, the explant is treated with a pesticide, for example by dipping said explant into a known concentration(s) of a pesticide containing solution or by spraying the explant with such a solution.

In a further embodiment of the invention the explant is sprayed immediately after being cut from a mother plant or, preferably, first grown for 1 to a few days before being sprayed. The so treated stem explants are then transferred to a suitable plant anchorage material, but are preferably planted in soil or placed in agar, and growth of the developing plantlet is monitored.

Owing to its easiness and simplicity, the method according to the invention is especially suitable to be used within a high through-put format.

### Examples

The following examples further describe the materials and methods used in carrying out the invention and the subsequent results. They are offered by way of illustration, and their recitation should not be considered as a limitation of the claimed invention.

### Example 1

A basal segment including a short root and stem are excised from 6 week old *A. myosuroides* plants growing in a controlled climate chamber (20°C day/16°C night; 12 hr photoperiod; 67% RH) in pots containing vermiculite (VERMEX exfolliiertes (expandiertes) Vermiculit- VERMICA AG) and watered with nutrient solution made from the commercially available soluble fertilizer [Hauert-Flory® 3 [HP-30], 1g/L] (water soluble fertilizer); N:P:K 1.5:1:1.5; Hauert & Co., 3257 Grossaffoltern].

The segments are transplanted into vermiculite containing HP-3 nutrient solution and placed in the climate chamber again. Nine days after transplanting the trays are sprayed in a spray chamber with one of the following herbicides- clodinafop 15 and 30g/ha, fenoxaprop 25 and 50g/ha, sethoxydim 150 and 300g/ha, isoproturon 500 and 1000g/ha, chlortoluron 500 and 1000g/ha.

Mortality (%) is assessed 3 weeks after spraying.

| HERBICIDE TREATMENT | BIOTYPES | | | |
|---|---|---|---|---|
| | S | R1 | R2 | R31 |
| Clodinafop (15g/ha) | 100 | 0 | 28 | 8 |
| Clodinafop (30g/ha) | 100 | 0 | 72 | 72 |
| Fenoxaprop (25g/ha) | 48 | 0 | 0 | 0 |
| Fenoxaprop (50g/ha) | 88 | 0 | 24 | 0 |
| Sethoxydim (150g/ha) | 100 | 0 | 100 | 100 |
| Sethoxydim (300g/ha) | 100 | 0 | 100 | 100 |
| Chlortoluron (1000g/ha) | 100 | 29 | 68 | 96 |
| Chlortoluron (500g/ha) | 100 | 14 | 8 | 88 |
| untreated | 0 | 0 | 0 | 0 |
| R1, R2, R3 are known resistant biotypes and S is a wild type (herbicide susceptible) | | | | |

### Example 2

Seeds from two resistant and one susceptible A. *myosuroides* biotypes are sown in_pots containing sterile potting loam in a temperature regulated glasshouse. Four weeks after sowing, 2-3 leafed plants are sprayed with one of the following herbicides- clodinafop 15 and 30g/ha, fenoxaprop 50 and 80g/ha, isoproturon 250 and 1000g/ha. Twelve days after spraying herbicide damage is recorded and a basal segment frcm surviving plants including a short root and stem are excised. One week after transplanting one of the following herbicides- clodinafop 20 and 40g/ha, fenoxaprop 80 and 120g/ha, isoproturon 500 and 1000g/ha. Two weeks after spraying herbicide damage is recorded (see below) Herbicide damage (%) is assessed 3 weeks after spraying.

| HERBICIDE TREATMENT | BIOTYPES | | |
|---|---|---|---|
| | R1 | R2 | S |
| Clodinafop (20g/ha) | 0 | 9 | 47 |
| Clodinafop (40g/ha) | 0 | 21 | 93 |
| Fenoxaprop (80g/ha) | 0 | 24 | 75 |
| Fenoxaprop (120g/ha) | 0 | 36 | 94 |
| Isoproturon (500g/ha) | 14 | 13 | 53 |
| Isoproturon (1000g/ha) | 54 | 53 | 92 |
| untreated | 0 | 0 | 0 |
| R1, R2 are known resistant biotypes and S is a wild type (herbicide susceptible) | | | |

### Example 3

A basal segment including a shoot and a short root are excised from field growing plants A. *myosuroides* and *Avena fatua* in the UK and from glasshouse growing herbicide resistant and susceptible A. *myosuroides* biotypes and planted in sterile loam in a glasshouse. Ten days after transplanting plants were sprayed with clodinafop (7.5, 15, 30 g ha⁻¹), fenoxaprop (13.8, 27.5, 55 g ha⁻¹) or isoproturon (250, 500, 1000 g ha⁻¹).

Herbicide damage (%) was recorded 13 days after spraying.

| | | | Biotype | | | | |
|---|---|---|---|---|---|---|---|
| Clodinafop | 1 | 2 | 3 4 | | 5 | 6 | 7 |
| 30 | 97 | 27 | 73 | 7 | 66 | 100 | 53 |
| 15 | 96 | 3 | 37 | 3 | 27 | 96 | 13 |
| 7.5 | 56 | 0 | 3 | 0 | 17 | 83 | 3 |

| Fenoxaprop | | | | | | | |
|---|---|---|---|---|---|---|---|
| 55 | 98 | 17 | 50 | 3 | 47 | 98 | 17 |
| 27.5 | 67 | 0 | 13 | 3 | 3 | 82 | 13 |
| 13.8 | 17 | 0 | 3 | 0 | 0 | 37 | 0 |

| Isoproturon | | | | | | | |
|---|---|---|---|---|---|---|---|
| 1000 | 100 | 100 | 98 | 96 | 100 | 97 | 87 |
| 500 | 99 | 98 | 89 | 93 | 94 | 77 | 68 |
| 250 | 98 | 57 | 37 | 50 | 67 | 7 | 27 |
| untreated | 0 | 0 | 0 | 0 | 0 | 0 | 0 |
| Biotype 1 is a glasshouse grown known wild type *A. myosuroides.* | | | | | | | |
| Biotypes 2, 3 and 7 are *A. myosuroides* confirmed resistant. | | | | | | | |
| Biotype 4 is a field collected reported resistant *A. myosuroides.* | | | | | | | |
| Biotypes 5 and 6 are field collected reported resistant and wild type, respectively *A*. *fatua*. | | | | | | | |

### Example 4

Field collections of the dicotyledonous weeds *Amaranthus* and *Chenopodium* are regenerated from cuttings.

*Chenopodium* plants 12cm high growing in a field are removed and roots placed in water. The stem is cut above the first leaves, leaving only the first nodes. A considerable part of the roots remained in the ground when the plants are removed. No additional roots are excised. Cuttings are transplanted in 10cm pots containing a sand loam, in a greenhouse. *Amaranthus* plants 15cm diameter are dug out of the field. The stems are cut into small sections containing 2 nodal regions. The older nodal region is buried in soil in pots (as for *Chenopodium)* and the younger exposed.

Two weeks after transplanting new growth is evident for cuttings from both species. *Chenopodium* (5-15cm high) and *Amaranthus* (2-9cm high). Plants are sprayed with a 500ml commercially available spray bottle with one of 5 concentrations of chlorsulfuron. Ten pumps per pot are applied. Percentage herbicide damage is visually assessed 3 weeks after treatment. The results show a good dose response to increasing herbicide concentration can be achieved even with dicotyledonous weeds.

| CHLORSULFURON | *Polygonum* | *Amaranthus* |
|---|---|---|
| | % herbicide damage | |
| Control | 0 | 0 |
| 200µm | 98 | 100 |
| 50µm | 80 | 100 |
| 12.5µm | 70 | 80 |
| 3.125µm | 20 | 50 |
| 0.78µm | 0 | 30 |

### Example 5

Seeds from resistant and a susceptible *A. myosuroides* biotypes are sown in pots containing sterile potting soil in a glasshouse. Eight weeks after sowing, a basal segment (cutting) from plants including a short root and stem are excised. One week after transplanting 5-10cm long new leaves had grown and plants are herbicide treated with a 500ml commercially available spray bottle with the below herbicides.

Dilutions from a 1ml (commercial herbicide formulation)/litre (tap water) is made. For example 1x (below is a 1ml/litre concentration which for the commercial formulation of cycloxydim is 0.61mM of cycloxydim). Five pumps per pot are applied. Percentage herbicide damage is visually assessed 2 weeks after treatment. The experiment shows that herbicide resistance can even be detected with such a simple spraying system.

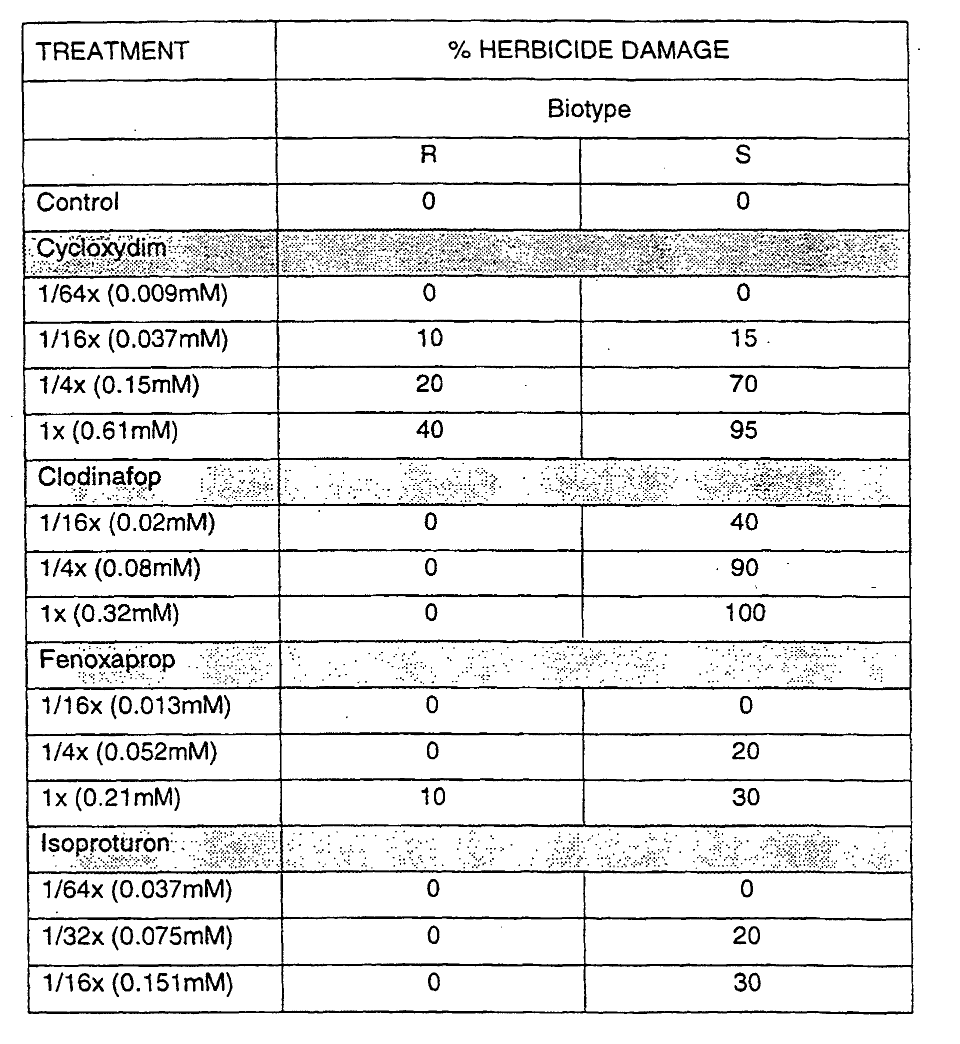

### Example 6

Young barley seedlings sprayed with test compounds in a cabinet sprayer and not showing powdery mildew symptoms (ie. the compounds are fungicidal) one week after inoculation are selected. A basal segment from plants including a short root and stem (cutting) is then excised and transplanted into pots containing sterile potting soil in a glasshouse. Two weeks after transplanting, new ieaf growth is evident and plants are either retreated with fungicide or not. Plants resprayed showed low fungal infection but plants not resprayed had very high fungal infection. This shows that cuttings from pretreated plants showed no lasting activity when inoculated 2 weeks after the first test indicating that none of the compounds are active in the plants after 2 weeks.

| *Fungicide compound* | *Plant background* | *Result (Fungal infection intensity)* | |
|---|---|---|---|
| *first fungicide treatment* | *second fungicide treatment* | *REPLICATE 1* | *REPLICATE 2* |
| NOA 405930 | NONE | 100% | 100% |
| (200 ppm ai) | Calixin | ∼ 40% | ∼ 40% |
| CGA 370142 | NONE | 100 % | 100% |
| (200 ppm ai) | Calixin | ∼ 40% | ∼ 40% |
| NONE | NONE | 100% | 100% |
| (Check) | Calixin | ∼ 40% | ∼ 40% |

### Example 7

Seeds from 4 resistant and a susceptible *A. myosuroides* biotypes are sown in pots containing sterile potting soil in a glasshouse. Eight weeks after sowing, a cutting comprising of a short root and stem is excised. The cuttings are dipped into one of various concentrations of the herbicide fenoxaprop and then immediately transplanted in pots containing sterile potting soil. Percentage herbicide damage is visually assessed 2 weeks after treatment. This experiment highlights the potential for not only spraying cuttings for resistance testing but also dipping the cuttings in herbicide or even inserting the cuttings into a medium already containing herbicide.

| *Biotypes* | *% VISUAL HERBICIDE DAMAGE* | | | |
|---|---|---|---|---|
| | 0 | 0.5mM fenoxaprop | 1 mM fenoxaprop | 2mM fenoxaprop |
| Susceptible | 0 | 80 | 90 | 98 |
| Resistant 1 | 0 | 0 | 0 | 5 |
| Resistant 2 | 0 | 40 | 50 | 60 |
| Resistant 3 | 0 | 15 | 25 | 40 |
| Resistant 4 | 0 | 20 | 50 | 60 |

## Claims

1. A method for testing progeny plants, comprising
(a) asexually propagating progeny plant(s) from a mother plant by cutting a segment from a mother plant, said segment comprising a meristematic region which is sufficiently intact to have retained its capability of regenerating a complete and morphologically normal plant, and directly regenerating said segment;
(b) incorporating the so obtained progeny plant into a plant screening program; and
(c) monitoring the growth of the progeny plant.

2. A method according to claim 1, wherein the propagation step (a) is accomplished by
(a) cutting a short segment from a mother plant such that said segment is capable of directly regenerating into a whole and morphologically normal plant,
(b) transferring said excised segment to a suitable anchorage material; and
(c) directly regenerating said transferred segment into a whole and morphologically normal plant.

3. A method according to claim 2, wherein said segment comprises a region that contains a high amount of actively dividing cells.

4. A method according to claim 3, wherein said region comprises meristematic cells.

5. A method according to claims 3 and 4, wherein said segment comprises a short root and shoot fragment.

6. A method according to claim 2, wherein the anchorage material is
(a) an inert material such as vermiculite, perlite or plastic beads;
(b) a culture medium commonly applied in plant cultivation; or
(c) soil.

7. A method according to any of the previous claims wherein said method is used within a high through-put format.

8. A method according to claim 1, wherein the propagation step (a) is accomplished by
(a) cutting a short segment from a mother plant such that said segment is capable of directly regenerating into a whole and morphologically normal plant;
(b)₁ dipping said segment into a known concentration(s) of a pesticide-containing solution; or, in the alternative
(b)₂ spraying said segment with a known concentration(s) of a pesticide- containing solution;
(c) transferring the so treated plant explants to a suitable anchorage material;
(d) directly regenerating said explant into a whole and morphologically normal plant.

9. Method of rescuing plants showing an interesting trait or property after treatment with a pesticide for further investigation comprising
(a) asexually propagating progeny plant(s) from a treated mother plant by cutting a segment from said mother plant, said segment comprising a meristematic region which is sufficiently intact to have retained its capability of regenerating a complete and morphologically normal plant, and directly regenerating said segment;
(b) incorporating the so obtained progeny plant into a plant screening program; and
(c) monitoring the growth of the progeny plant.

10. Method according to claim 9, wherein the propagation step (a) is accomplished by
(a) cutting a short segment from a treated mother plant such that said segment is capable of directly regenerating into a whole and morphologically normal plant;
(b) transferring said excised segment to a suitable anchorage material; and
(c) directly regenerating said transferred segment into a whole and morphologically normal plant.

11. Method according to claim 9, wherein the propagation step (a) is accomplished by
(a) cutting a short segment from a treated mother plant such that said segment is capable of directly regenerating into a whole and morphologically normal plant;
(b)₁ dipping said segment into a known concentration(s) of a pesticide-containing solution; or, in the alternative
(b)₂ spraying said segment with a known concentration(s) of a pesticide-containing solution;
(c) transferring the so treated plant explants to a suitable anchorage material;
(d) directly regenerating said explant into a whole and morphologically normal plant.

12. Method according to any one of claims 9 to 11 further comprising the step of further investigation of the rescued material.

13. Method according to any of the previous claims, wherein the pesticide is selected from the group consisting of a herbicide, an insecticide and a fungicide.

14. Method for determining whether a resistance phenotype observed in a plant is due to a resistance trait or caused by other factors, comprising
(a) collecting the phenotypically resistant plant
(b) asexually propagating progeny plant(s) from said resistant plant by cutting a segment from said resistant plant, said segment comprising a meristematic region which is sufficiently intact to have retained its capability of regenerating a complete and morphologically normal plant, and directly regenerating said segment;
(c) incorporating the so obtained progeny plant into a plant screening program; and
(d) monitoring the growth of the progeny plant.

15. Method according to claim 14, wherein the propagation step (b) is accomplished by
(a) cutting a short segment from said plant such that said segment is capable of directly regenerating into a whole and morphologically normal plant,
(b)₁ dipping said segment into a known concentration(s) of a pesticide-containing solution; or, in the alternative
(b)₂ spraying said segment with a known concentration(s) of a pesticide-containing solution;
(c) transferring the so treated plant explants to a suitable anchorage material;
(f) directly regenerating said explant into a whole and morphologically normal plant.

16. Method according to claims 14 and 15, wherein the resistance phenotype is observed after treating the plant with a pesticide.

17. Method according to claim 16, wherein the pesticide is selected from the group consisting of a herbicide, an insecticide and a fungicide.

18. Method according to any of the preceding claims, wherein the plant to be tested is a weed plant.

19. Method according to any of the preceding claims, wherein the plant to be tested is a crop plant.

20. Method according to any of the preceding claims, wherein the plant to be tested is a transgenic plant.

## Patentansprüche

1. Verfahren zum Testen von Nachkommenpflanzen, umfassend
(a) asexuelles Fortpflanzen einer Nachkommenpflanze (von Nachkommenpflanzen) aus einer Mutterpflanze durch Schneiden eines Segments aus einer Mutterpflanze, wobei das Segment eine meristematische Region umfasst, die ausreichend intakt ist, um ihre Fähigkeit, eine vollständige und morphologisch normale Pflanze zu regenerieren, beibehalten zu haben, und direktes Regenerieren des Segments;
(b) Einbauen der so erhaltenen Nachkommenpflanze in ein Pflanzen-Screening-Programm und
(c) Überwachen des Wachstums der Nachkommenpflanze.

2. Verfahren nach Anspruch 1, wobei die Fortpflanzungsstufe (a) ausgeführt wird durch
(a) Schneiden eines kurzen Segments aus einer Mutterpflanze derart, dass das Segment fähig ist, direkt zu einer ganzen und morphologisch normalen Pflanze zu regenerieren;
(b) Transferieren des ausgeschnittenen Segments auf ein geeignetes Verankerungsmaterial; und
(c) direktes Regenerieren des transferierten Segments zu einer ganzen und morphologisch normalen Pflanze.

3. Verfahren nach Anspruch 2, wobei das Segment eine Region umfasst, die eine große Menge an sich aktiv teilenden Zellen enthält.

4. Verfahren nach Anspruch 3, wobei die Region meristematische Zellen umfasst.

5. Verfahren nach Anspruch 3 und 4, wobei das Segment ein kurzes Wurzel- und Schößlingfragment umfasst.

6. Verfahren nach Anspruch 2, wobei das Verankerungsmaterial
(a) ein inertes Material, z.B. Vermiculit-, Perlitoder Kunststoffperlen ist;
(b) ein Kulturmedium, das üblicherweise bei der Pflanzenkultivierung angewendet wird, oder
(c) Erde
ist.

7. Verfahren nach einem der vorangehenden Ansprüche, wobei das Verfahren in einem Format mit hohem Durchsatz verwendet wird.

8. Verfahren nach Anspruch 1, wobei die Fortpflanzungsstufe
(a) durchgeführt wird durch
(a) Schneiden eines kurzen Segments aus einer Mutterpflanze derart, dass das Segment fähig ist, direkt zu einer ganzen und morphologisch normalen Pflanze zu regenerieren;
(b)₁ Eintauchen des Segments in eine bekannte Konzentration (in bekannte Konzentrationen) einer Pestizid enthaltenden Lösung; oder alternativ
(b)₂ Besprühen des Segments mit einer bekannten Konzentration (mit bekannten Konzentrationen) einer Pestizid enthaltenden Lösung;
(c) Transferieren der so behandelten Pflanzenexplantate in ein geeignetes Verankerungsmaterial;
(d) direktes Regenerieren des Explantats zu einer ganzen und morphologisch normalen Pflanze.

9. Verfahren zum Retten von Pflanzen, die nach Behandlung mit einem Pestizid ein interessantes Merkmal oder eine interessante Eigenschaft zeigen, zur weiteren Untersuchung, umfassend
(a) asexuelles Fortpflanzen einer Nachkommenpflanze / von Nachkommenpflanzen aus einer behandelten Mutterpflanze durch Schneiden eines Segments aus der Mutterpflanze, wobei das Segment eine meristematische Region umfasst, die ausreichend intakt ist, um ihre Fähigkeit, eine vollständige und morphologisch normale Pflanze zu regenerieren, beibehalten zu haben, und direktes Regenerieren des Segments;
(b) Einbauen der so erhaltenen Nachkommenpflanze in ein Pflanzen-Screening-Programm und
(c) Überwachen des Wachstums der Nachkommenpflanze.

10. Verfahren nach Anspruch 9, wobei die Fortpflanzungsstufe (a) ausgeführt wird durch
(a) Schneiden eines kurzen Segments aus einer behandelten Mutterpflanze derart, dass das Segment fähig ist, direkt zu einer ganzen und morphologisch normalen Pflanze zu regenerieren;
(b) Transferieren des ausgeschnittenen Segments auf ein geeignetes Verankerungsmaterial; und
(c) direktes Regenerieren des transferierten Segments zu einer ganzen und morphologisch normalen Pflanze.

11. Verfahren nach Anspruch 9, wobei die Fortpflanzungsstufe (a) ausgeführt wird durch
(a) Schneiden eines kurzen Segments aus einer behandelten Mutterpflanze derart, dass das Segment fähig ist, direkt zu einer ganzen und morphologisch normalen Pflanze zu regenerieren;
(b)₁ Eintauchen des Segments in eine bekannte Konzentration (in bekannte Konzentrationen) einer Pestizid enthaltenden Lösung; oder alternativ
(b)₂ Besprühen des Segments mit einer bekannten Konzentration (mit bekannten Konzentrationen) einer Pestizid enthaltenden Lösung;
(c) Transferieren der so behandelten Pflanzenexplantate auf ein geeignetes Verankerungsmaterial;
(d) direktes Regenerieren des Explantats zu einer ganzen und morphologisch normalen Pflanze.

12. Verfahren nach einem der Ansprüche 9 bis 11, das außerdem die Stufe einer weiteren Untersuchung des geretteten Materials umfasst.

13. Verfahren nach einem der vorangehenden Ansprüche, wobei das Pestizid ausgewählt wird aus der Gruppe, bestehend aus einem Herbizid, einem Insektizid und einem Fungizid.

14. Verfahren zur Bestimmung, ob ein bei einer Pflanze beobachteter Resistenzphänotyp auf einem Resistenzmerkmal beruht oder durch andere Faktoren verursacht ist, umfassend:
(a) Sammeln der phänotypisch resistenten Pflanze;
(b) asexuelles Fortpflanzen einer Nachkommenpflanze (von Nachkommenpflanzen) aus der resistenten Pflanze durch Schneiden eines Segments aus der resistenten Pflanze, wobei das Segment eine meristematische Region umfasst, die ausreichend intakt ist, um ihre Fähigkeit, eine vollständige und morphologisch normale Pflanze zu regenerieren, beibehalten zu haben, und direktes Regenerieren des Segments;
(c) Einbauen der so erhaltenen Nachkommenpflanze in ein Screening-Programm und
(d) Überwachen des Wachstums der Nachkommenpflanze.

15. Verfahren nach Anspruch 14, wobei die Fortpflanzungsstufe (b) ausgeführt wird durch
(a) Schneiden eines kurzen Segments aus der Pflanze derart, dass das Segment fähig ist, direkt zu einer ganzen und morphologisch normalen Pflanze zu regenerieren;
(b)₁ Eintauchen des Segments in eine bekannte Konzentration (in bekannte Konzentrationen) einer Pestizid enthaltenden Lösung oder alternativ
(b)₂ Besprühen des Segments mit einer bekannten Konzentration (bekannten Konzentrationen) einer Pestizid enthaltenden Lösung;
(c) Transferieren der so behandelten Pflanzenexplantate auf ein geeignetes Verankerungsmaterial;
(f) direktes Regenerieren des Explantats zu einer ganzen und morphologisch neutralen Pflanze.

16. Verfahren nach Anspruch 14 und 15, wobei der Resistenzphänotyp nach Behandlung der Pflanze mit einem Pestizid beobachtet wird.

17. Verfahren nach Anspruch 16, wobei das Pestizid aus der Gruppe, bestehend aus einem Herbizid, einem Insektizid und einem Fungizid, ausgewählt wird.

18. Verfahren nach einem der vorangehenden Ansprüche, wobei die zu testende Pflanze eine Unkrautpflanze ist.

19. Verfahren nach einem der vorangehenden Ansprüche, wobei die zu testende Pflanze eine Erntefruchtpflanze ist.

20. Verfahren nach einem der vorangehenden Ansprüche, wobei die zu testende Pflanze eine transgene Pflanze ist.

## Revendications

1. Méthode utilisée pour tester des descendants de plantes, comprenant les étapes consistant à
(a) propager de manière asexuelle un (des) descendant(s) de plantes à partir d'une plante mère en coupant un segment à partir d'une plante mère, ledit segment comprenant une région méristématique qui est suffisamment intacte pour avoir conservé sa capacité à régénérer une plante complète et normale sur le plan morphologique, et à régénérer directement ledit segment ;
(b) incorporer le descendant de la plante ainsi obtenu dans un programme de sélection de plantes ; et
(c) surveiller la croissance du descendant de la plante.

2. Méthode selon la revendication 1, dans laquelle l'étape de propagation (a) est réalisée en
(a) coupant un segment de petite taille à partir d'une plante mère de telle manière que ledit segment soit capable de se régénérer directement dans une plante entière et normale sur le plan morphologique,
(b) transférer ledit segment excisé dans un matériau d'ancrage adéquat ; et
(c) régénérer directement ledit segment transféré dans une plante entière et normale sur le plan morphologique.

3. Méthode selon la revendication 2, dans laquelle ledit segment comprend une région qui contient une quantité élevée de cellules se divisant activement.

4. Méthode selon la revendication 3, dans laquelle ladite région comprend des cellules méristématiques.

5. Méthode selon les revendications 3 et 4, dans laquelle ledit segment comprend un fragment d'ancrage de petite taille.

6. Méthode selon la revendication 2, dans laquelle le matériau d'ancrage est
(a) un matériau inerte tel que de la vermiculite, de la perlite ou des billes en plastique ;
(b) un milieu de culture généralement appliqué à la culture des plantes ; ou
(c) de la terre.

7. Méthode selon l'une quelconque des revendications précédentes, dans laquelle ladite méthode est utilisée dans un format à haut débit.

8. Méthode selon la revendication 1, dans laquelle l'étape de propagation (a) est réalisée en
(a) coupant un segment de petite taille à partir d'une plante mère de telle manière que ledit segment soit capable de se régénérer directement dans une plante entière et normale sur le plan morphologique ;
(b)₁ plongeant ledit segment dans une solution contenant des pesticides de concentration(s) connue(s) ; ou, en variante
(b)₂ pulvérisant ledit segment avec une solution contenant des pesticides de concentration(s) connue(s) ;
(c) transférant les explants issus de plantes ainsi traités dans un matériau d'ancrage adéquat ;
(d) régénérant directement ledit explant dans une plante entière et normale sur le plan morphologique.

9. Méthode de récupération de plantes montrant un trait intéressant ou une propriété intéressante après traitement avec un pesticide, destinée à une étude complémentaire comprenant les étapes consistant à
(a) propager de manière asexuelle un (des) descendant(s) de plantes à partir d'une plante mère traitée en coupant un segment à partir de ladite plante mère, ledit segment comprenant une région méristématique qui est suffisamment intacte pour avoir conservé sa capacité à régénérer une plante complète et normale sur le plan morphologique, et à régénérer directement ledit segment ;
(b) incorporer le descendant de la plante ainsi obtenu dans un programme de sélection de plantes ; et
(c) surveiller la croissance du descendant de la plante.

10. Méthode selon la revendication 9, dans laquelle l'étape de propagation (a) est réalisée en
(a) coupant un segment de petite taille à partir d'une plante mère traitée de telle manière que ledit segment soit capable de se régénérer directement dans une plante entière et normale sur le plan morphologique ;
(b) transférer ledit segment excisé dans un matériau d'ancrage adéquat ; et
(c) régénérer directement ledit segment transféré dans une plante entière et normale sur le plan morphologique.

11. Méthode selon la revendication 9, dans laquelle l'étape de propagation (a) est réalisée en
(a) coupant un segment de petite taille à partir d'une plante mère traitée de telle manière que ledit segment soit capable de se régénérer directement dans une plante entière et normale sur le plan morphologique ;
(b)₁ plongeant ledit segment dans une solution contenant des pesticides de concentration(s) connue(s) ; ou, en variante
(b)₂ pulvérisant ledit segment avec une solution contenant des pesticides de concentration(s) connue(s) ;
(c) transférant les explants issus de plantes ainsi traités dans un matériau d'ancrage adéquat ;
(d) régénérant directement ledit explant dans une plante entière et normale sur le plan morphologique.

12. Méthode selon l'une quelconque des revendications 9 à 11, comprenant en outre l'étape d'étude complémentaire du matériau récupéré.

13. Méthode selon l'une quelconque des revendications précédentes, dans laquelle le pesticide est choisi dans le groupe consistant en un herbicide, un insecticide et un fongicide.

14. Méthode utilisée pour déterminer si un phénotype montrant une résistance observé dans une plante est dû à un trait de résistance ou est causé par d'autres facteurs, comprenant les étapes consistant à
(a) collecter la plante résistante sur le plan du phénotype
(b) propager de manière asexuelle un (des) descendant(s) de plantes à partir de ladite plante résistante en coupant un segment à partir de ladite plante résistante, ledit segment comprenant une région méristématique qui est suffisamment intacte pour avoir conservé sa capacité à régénérer une plante complète et normale sur le plan morphologique, et à régénérer directement ledit segment ;
(c) incorporer le descendant de la plante ainsi obtenu dans un programme de sélection de plantes ; et
(d) surveiller la croissance du descendant de la plante.

15. Méthode selon la revendication 14, dans laquelle l'étape de propagation (b) est réalisée en
(a) coupant un segment de petite taille à partir de ladite plante de telle manière que ledit segment soit capable de se régénérer directement dans une plante entière et normale sur le plan morphologique,
(b)₁ plongeant ledit segment dans une solution contenant des pesticides de concentration(s) connue(s) ; ou, en variante
(b)₂ pulvérisant ledit segment avec une solution contenant des pesticides de concentration(s) connue(s) ;
(c) transférant les explants issus de plantes ainsi traités dans un matériau d'ancrage adéquat ;
(f) régénérant directement ledit explant dans une plante entière et normale sur le plan morphologique.

16. Méthode selon la revendication 14 et 15, dans laquelle le phénotype montrant une résistance est observé après avoir traité la plante avec un pesticide.

17. Méthode selon la revendication 16, dans laquelle le pesticide est choisi dans le groupe consistant en un herbicide, un insecticide et un fongicide.

18. Méthode selon l'une quelconque des revendications précédentes, dans laquelle la plante devant être testée est une mauvaise herbe.

19. Méthode selon l'une quelconque des revendications précédentes, dans laquelle la plante devant être testée est une plante cultivée.

20. Méthode selon l'une quelconque des revendications précédentes, dans laquelle la plante devant être testée est une plante transgénique.
